# EUROPÄISCHE PATENTANMELDUNG

(11) **EP 0 676 190 A2**
(43) Veröffentlichungstag der Anmeldung: **11.10.1995**
(21) Anmeldenummer: 95103611.0
(22) Anmeldetag: 14.03.1995
(51) Int. Cl.: A61K 7/32

(54) **Gegen Fussgeruch wirksame Zubereitungen mit einem Gehalt an Monoglycerinestern von Fettsäuren**

(30) Priorität: 07.04.1994 DE 4411899
(71) Anmelder: Beiersdorf Aktiengesellschaft, D-20245 Hamburg (DE)
(72) Erfinder: Wolf, Florian, Dr., D-20245 Hamburg (DE); Traupe, Bernd, D-22457 Hamburg (DE); Klier, Manfred, Dr., D-20521 Aumühle (DE)

(57) **Zusammenfassung**

Verwendung von Glyceriden und/oder Oligoglyceriden verzweigter und/oder unverzweigter gesättigter und/oder ungesättigter Monocarbonsäuren mit 8 - 14 Kohlenstoffatomen zur Prophylaxe oder Bekämpfung des durch die Zersetzung des menschlichen Fußschweißes durch Brevibakterien hervorgerufenen Fußgeruches.

## Beschreibung

Die vorliegende Erfindung betrifft Wirkstoffe und Zubereitungen, solche Wirkstoffe enthaltend, welche gegen Fußgeruch wirksam sind.

Mikroorganismen im allgemeinen, aber auch Bakterien im besonderen, sind praktisch allgegenwärtig. Auf der gesunden menschlichen Haut beispielsweise sind hauptsächlich Mycobakterien, Streptokokken, Staphylokokken und Propionibakterien zu finden.

Ebenfalls auf der Haut vorkommende coryneforme Bakterien werden neuerdings für die Entstehung unangenehmen Körpergeruches durch die Zersetzung apokrinen Schweißes verantwortlich gemacht.

Verursacher des typisch käseartigen Geruches verschwitzter Füße ist Brevibacterium spec.

Brevibakterien sind ebenfalls coryneforme Bakterien. Es handelt sich bei ihnen um fakultativ anaerobe, grampositive, zylinderförmige Bakterien. Der durch den Befall der Füße mit Brevibacterium spec. verursachte "käsige" Geruch kommt nicht von ungefähr, da bei der Reifung mancher Käse, insbesondere Weichkäse, Brevibakterien eine Rolle spielen (z.B. Brevibacterium linens).

Fußgeruch wird für gewöhnlich mit herkömmlichen Desodorantien bzw. Antitranspirantien, in Analogie zum Achselgeruch, oder auch einfach nur mit Wasser und Seife bekämpft.

In Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Fußregion ein und sollten allenfalls in Ausnahmefällen angewandt werden.

Durch die Verwendung antimikrobieller Stoffe kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Fußgeruch auch durch Duftstoffe überdeckt werden, eine Methode, die am wenigsten den ästhetischen Bedürfnissen des Verbrauchers gerecht wird, da die Mischung aus Fußgeruch und Parfümduft eher unangenehm riecht.

Allerdings werden die meisten Kosmetika, parfümiert, auch wenn sie antimikrobiell wirksame Stoffe enthalten. Parfümierung kann auch dazu dienen, die Verbraucherakzeptanz eines kosmetischen Produktes zu erhöhen oder einem Produkt ein bestimmtes Flair zu geben.

Zwar sind existiert eine Fülle an antimikrobiell wirksamen Substanzen, insbesondere auch solcher Substanzen, welche "in vitro" gegen Brevibakterien wirksam sind. "In vivo", also auf dem menschlichen Fuße, besiedeln jedoch Brevibakterien tiefer gelegene Hautschichten, welche von den weitaus meisten antimikrobiell wirksamen Substanzen gar nicht erreicht werden.

Es genügt daher in aller Regel nicht, einen physiologisch verträglichen antimikrobiell wirksamen Stoff auf die von Brevibakterien befallene Fußhaut aufzutragen.

Es war also die Aufgabe der vorliegenden Erfindung, Zubereitungen zur Verfügung zu stellen, bei deren Verwendung es möglich ist, den durch die Zersetzung des menschlichen Fußschweißes durch Brevibakterien hervorgerufenen Fußgeruch "in vivo" zu bekämpfen bzw. seiner Entstehung zuvorzukommen.

Die Lösung der Aufgabe besteht in der
Verwendung von Glyceriden verzweigter und/oder unverzweigter gesättigter und/oder ungesättigter Monocarbonsäuren mit 8 - 14 Kohlenstoffatomen zur Prophylaxe oder Bekämpfung des durch die Zersetzung des menschlichen Fußschweißes durch Brevibakterien hervorgerufenen Fußgeruches.

Erstaunlicherweise penetrieren die erfindungsgemäßen Monoglyceride die Fußhaut und bekämpfen dort selektiv die für den Fußgeruch verantwortlichen Brevibakterien.

Zwar ist bekannt, daß Fettsäureester des Glycerins gewisse antimikrobielle Wirkung zeitigen. Bekannt ist ferner, Glycerinfettsäureester in desodorierenden Kosmetika einzusetzen. Der einzige Fettsäureester, der wirklich als praktikabel galt, ist jedoch das Glycerinmonolaurat.

Ferner ist aus der JP-OS Sho-48/019940 der Taiyo Kagaku Kogyo Co. Ltd. ein Kosmetikum mit antiseptischen Eigenschaften bekannt, welches sich durch einen Gehalt an Caprylsäuremonoglycerid auszeichnet.

Schließlich werden in der EP-OS 530 861 topische antimikrobielle pharmazeutische Kombinationen beschrieben, enthaltend neben Glycerinmonocaprylat und Glycerinmonocaprinat auch ein Gemisch aus gesättigten Fettsäuren.

Erfindungsgemäß werden die Monoglyceride mittellanger Fettsäuren durch die allgemeine Formel 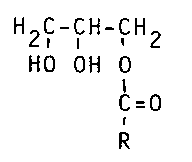
bzw. 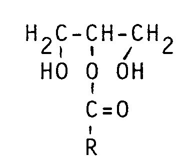
wiedergegeben, wobei R einen verzweigten oder unverzweigten Alkyl- oder Alkenylrest von 7 bis 13 C-Atomen darstellt.

Besonders vorteilhaft stellt R den Octanoylrest (Caprylsäurerest) bzw. den Decanoylrest (Caprinsäurerest) dar, wird also durch die Formeln 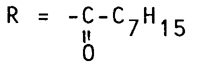
bzw. 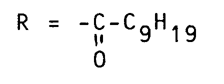
repräsentiert.

In dieser Schrift, insbesondere in den Beispielen, wird das Kürzel GMCy für Glycerinmonocaprylat und das Kürzel GMC für Glycerinmonocaprinat verwendet.

Bei den in 1-Position des Glycerins veresterten Glycerinestern ist die 2-Position ein Asymmetriezentrum. Erfindungsgemäß aktiv und gleichermaßen von Vorteil sind die 2S- und die 2R-Konfiguration.

Es hat sich als günstig herausgestellt, racemische Gemische der Stereoisomeren zu verwenden.

Die erfindungsgemäßen kosmetischen Zubereitungen gegen Fußgeruch sind besonders vorteilhaft dadurch gekennzeichnet, daß die erfindungsgemäßen Monoglycerinester in Konzentrationen von 0,05 - 10,00 Gew.-%, bevorzugt 0,1 - 5,0 Gew.-%, vorliegen, jeweils bezogen auf das Gesamtgewicht der Zubereitungen.

Die erfindungsgemäßen gegen unreine Haut wirksamen Zubereitungen können in Form von mittels Pinseln oder Abstreifern oder Roll-on-Vorrichtungen auftragbaren flüssigen Zusammensetzungen, als Stifte und in Form von aus normalen Flaschen und Behältern auftragbaren W/O- oder O/W-Emulsionen, z.B. Crèmes oder Lotionen.

Als übliche Trägerstoffe zur Herstellung der erfindungsgemäßen gegen unreine Haut wirksamen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Emulgatoren zur Herstellung der erfindungsgemäßen gegen Fußgeruch wirksamen Zubereitungen, welche vorteilhaft als flüssige Zubereitungen auf die Füße aufgetragen werden sollen, vorteilhaft mittels eines Wattebausches, und die in den Zubereitungen in geringer Menge, z.B. 2 bis 5 Gewichts.-%, bezogen auf die Gesamt-Zusammensetzung, verwendet werden können, haben sich nichtionogene Typen, wie Polyoxyethylen-Fettalkoholether, z.B. Cetylstearylalkoholpolyethylenglykolether mit 12 bzw. 20 angelagerten Ethylenoxid-Einheiten pro Molekül, Cetylstearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen Zubereitungen, deren pH-Wert vorzugsweise z.B. durch übliche Puffergemische auf 4,0 bis 9,0 insbesondere 5,0 bis 6,5, eingestellt wird, Parfüm, Farbstoffe, Antioxidantien (z.B. α-Tocopherol und seine Derivate oder Butylhydroxytoluol (BHT = 2,6-Di-tert.-butyl-4-methylphenol) in Mengen von 0,01 bis 0,03 %, bezogen auf die Gesamtzusammensetzung), Suspendiermittel, Puffergemische oder andere übliche kosmetische Grundstoffe beigemischt werden.

Vorteilhaft wird der pH-Wert der erfindungsgemäßen Zubereitungen im schwach sauren bis neutralen Bereich eingestellt, bevorzugt von 4,0 - 7,0 , besonders bevorzugt von 5,0 - 6,5.

Die jeweils einzusetzenden Mengen an Trägerstoffen können in Abhängigkeit von der Art des jeweiligen Produktes vom Fachmann durch einfaches Ausprobieren leicht ermittelt werden.

Die Herstellung der erfindungsgemäßen Zubereitungen erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Es folgen vorteilhafte Ausführungsbeispiele der vorliegenden Erfindung.

### Beispiel 1

### Aerosolspray

| (a) Flüssige Phase | |
|---|---|
| | Gew.-% |
| Octyldodecanol | 0,50 |
| GMCy | 0,30 |
| Parfüm | q.s. |
| Ethylalkohol | ad 100,00 |
| (b) Die unter (a) erhaltene flüssige Phase wird zusammen mit einem Propan/Butan 2,7 - Gemisch im Verhältnis 39 : 61 in Aerosolbehälter abgefüllt. | |

### Beispiel 2

### Pumpspray

| (a) | |
|---|---|
| | Gew.-% |
| Ethylalkohol | 60,00 |
| Glycerin | 1,00 |
| PEG-40-Hydriertes Ricinusöl | 2,00 |
| GMCy | 0,70 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 3

### Roll on - Gel

| | Gew.-% |
|---|---|
| | |

| (a) | |
|---|---|
| 1,3-Butylenglycol | 2,00 |
| Hydroxyethylcellulose | 0,50 |
| (z.B. Tylose 4000, Hoechst) | |

| (b) | |
|---|---|
| Ethylalkohol | 60,00 |
| PEG-40-Hydriertes Ricinusöl | 2,00 |
| GMCy | 0,80 |
| Parfum | q.s. |

| (c) | |
|---|---|
| Wasser | ad 100,00 |

Die unter (a) genannten Bestandteile werden dispergiert, Wasser (c) wird zugegeben, bei Raumtemperatur quellen gelassen, eine Lösung der unter (b) genannten Bestandteile wird nach ca. 15 Minuten zugegeben. Die entstanden Mischung wird homogenisiert und kann abgefüllt werden.

### Beispiel 5

### Wachsstift

| | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| GMCy | 0,70 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 6

### Roll on - Emulsion

| (a) | |
|---|---|
| | Gew.-% |
| Tricetearethphosphat | 0,30 |
| Octyldodecanol | 2,00 |
| C₁₂₋₁₅-Alkohol-Benzoate | 2,00 |
| GMCy | 0,90 |
| C₁₀₋₃₀-Alkylacrylate | 0,15 |

| (b) | |
|---|---|
| Ethylalkohol | 10,00 |
| Parfum | q.s. |

| (c) | |
|---|---|
| NaOH | 0,05 |
| Wasser | ad 100,00 |

Die unter (a) und (c) genannten Bestandteile werden jeweils unter Rühren auf 75° C erwärmt. Sodann werden die Bestandteile (a) zu (c) gegeben. Die Mischung wird auf 35° C abgekühlt. Aus den Bestandteilen (b) wird eine Lösung hergestellt, welche auf 35° C erwärmt wird und unter Rühren zur Mischung aus (a) und (c) gegeben wird.

### Beispiel 7

### Aerosolspray

| (a) Flüssige Phase | |
|---|---|
| | Gew.-% |
| Octyldodecanol | 0,50 |
| GMC | 0,65 |
| Parfüm | q.s. |
| Ethylalkohol | ad 100,00 |
| (b) Die unter (a) erhaltene flüssige Phase wird zusammen mit einem Propan/Butan 2,7 - Gemisch im Verhältnis 39 : 61 in Aerosolbehälter abgefüllt. | |

### Beispiel 9

### Pumpspray

| (a) | |
|---|---|
| | Gew.-% |
| Ethylalkohol | 60,00 |
| Glycerin | 1,00 |
| PEG-40-Hydriertes Ricinusöl | 2,00 |
| GMC | 0,70 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 10

### Roll on - Gel

| | Gew.-% |
|---|---|
| | |

| (a) | |
|---|---|
| 1,3-Butylenglycol | 2,00 |
| Hydroxyethylcellulose | 0,50 |
| (z.B. Tylose 4000, Hoechst) | |

| (b) | |
|---|---|
| Ethylalkohol | 60,00 |
| PEG-40-Hydriertes Ricinusöl | 2,00 |
| GMC | 0,90 |
| Parfum | q.s. |

| (c) | |
|---|---|
| Wasser | ad 100,00 |

Die unter (a) genannten Bestandteile werden dispergiert, Wasser (c) wird zugegeben, bei Raumtemperatur quellen gelassen, eine Lösung der unter (b) genannten Bestandteile wird nach ca. 15 Minuten zugegeben. Die entstanden Mischung wird homogenisiert und kann abgefüllt werden.

### Beispiel 11

### Wachsstift

| | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| GMC | 1,25 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 12

### Roll on - Emulsion

| (a) | |
|---|---|
| | Gew.-% |
| Tricetearethphosphat | 0,30 |
| Octyldodecanol | 2,00 |
| C₁₂₋₁₅-Alkohol-Benzoate | 2,00 |
| GMC | 1,10 |
| C₁₀₋₃₀-Alkylacrylate | 0,15 |

| (b) | |
|---|---|
| Ethylalkohol | 10,00 |
| Parfum | q.s. |

| (c) | |
|---|---|
| NaOH | 0,05 |
| Wasser | ad 100,00 |

Die unter (a) und (c) genannten Bestandteile werden jeweils unter Rühren auf 75° C erwärmt. Sodann werden die Bestandteile (a) zu (c) gegeben. Die Mischung wird auf 35° C abgekühlt. Aus den Bestandteilen (b) wird eine Lösung hergestellt, welche auf 35° C erwärmt wird und unter Rühren zur Mischung aus (a) und (c) gegeben wird.

## Patentansprüche

1. Verwendung von Glyceriden verzweigter und/oder unverzweigter gesättigter und/oder ungesättigter Monocarbonsäuren mit 8 - 14 Kohlenstoffatomen zur Prophylaxe oder Bekämpfung des durch die Zersetzung des menschlichen Fußschweißes durch Brevibakterien hervorgerufenen Fußgeruches.

2. Verwendung nach Anspruch 1, dadurch gekennzeichnet, daß die Glyceride mittellanger Fettsäuren durch die allgemeine Formel 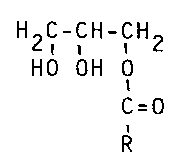 bzw. 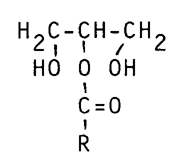 wiedergegeben werden, wobei R einen verzweigten oder unverzweigten Alkyl- oder Alkenylrest von 7 bis 13 C-Atomen darstellt.
